# EUROPÄISCHE PATENTANMELDUNG

(11) **EP 3 267 235 A1**
(43) Veröffentlichungstag der Anmeldung: **10.01.2018**
(21) Anmeldenummer: 17177112.4
(22) Anmeldetag: 21.06.2017
(51) Int. Cl.: G02B 23/24

(54) **OPTISCHES SYSTEM EINES STEREO-VIDEOENDOSKOPS, STEREO-VIDEOENDOSKOP UND VERFAHREN ZUM BETREIBEN EINES OPTISCHEN SYSTEMS EINES STEREO-VIDEOENDOSKOPS**

(30) Priorität: 08.07.2016 DE 102016212470
(71) Anmelder: OLYMPUS Winter & Ibe GmbH, 22045 Hamburg (DE)
(72) Erfinder: ZHAO, Jianxin, 22399 Hamburg (DE)
(74) Vertreter: Seemann & Partner Patentanwälte mbB

(57) **Zusammenfassung**

Die Erfindung betrifft ein optisches System (40) eines Stereo-Videoendoskops (2), Stereo-Videoendoskop (2) und Verfahren zum Betreiben eines optischen Systems (40). Das optische System (40) umfasst eine distale optische Baugruppe (42) und eine proximale optische Baugruppe (44) mit einem linken Linsensystemkanal (18a) und einem rechten Linsensystemkanal (18b). Die distale optische Baugruppe (42) koppelt aus einem Objektraum (26) einfallendes Licht in den linken Linsensystemkanal (18a) und in den rechten Linsensystemkanal (18b) der proximalen optischen Baugruppe (44) ein. Die distale optische Baugruppe (42) ist eine optische Baugruppe mit veränderlicher Brennweite, wobei eine Veränderung der Brennweite eine Verschiebung eines Achsenkreuzungspunkts (24) im Objektraum (26) bewirkt.

## Beschreibung

Die Erfindung betrifft ein optisches System eines Stereo-Videoendoskops, umfassend eine distale optische Baugruppe und eine proximale optische Baugruppe, wobei die proximale optische Baugruppe einen linken Linsensystemkanal mit einer linken optischen Achse und einen rechten Linsensystemkanal mit einer rechten optischen Achse umfasst, und wobei der linke und der rechte Linsensystemkanal gleichartig aufgebaut und die linke und die rechte optische Achse parallel zueinander ausgerichtet sind, und wobei die distale optische Baugruppe dazu eingerichtet ist, aus einem Objektraum einfallendes Licht in den linken Linsensystemkanal und in den rechten Linsensystemkanal der proximalen optischen Baugruppe einzukoppeln, und wobei die distale optische Baugruppe einen Achsenkreuzungspunkt im Objektraum festlegt, in dem sich eine linke Blickachse des linken Linsensystemkanals und eine rechte Blickachse des rechten Linsensystemkanals schneiden.

Ferner betrifft die Erfindung ein Stereo-Videoendoskop mit einem solchen optischen System sowie ein Verfahren zum Betreiben eines optischen Systems eines Stereo-Videoendoskops, wobei das optische System eine distale optische Baugruppe und eine proximale optische Baugruppe umfasst, und wobei die proximale optische Baugruppe einen linken Linsensystemkanal mit einer linken optischen Achse und einen rechten Linsensystemkanal mit einer rechten optischen Achse umfasst, und wobei der linke und der rechte Linsensystemkanal gleichartig aufgebaut und die linke und die rechte optische Achse parallel zueinander ausgerichtet sind, und wobei die distale optische Baugruppe aus einem Objektraum einfallendes Licht in den linken Linsensystemkanal und in den rechten Linsensystemkanal der proximalen optischen Baugruppe einkoppelt, und wobei die distale optische Baugruppe einen Achsenkreuzungspunkt im Objektraum festlegt, in dem sich eine linke Blickachse des linken Linsensystemkanals und eine rechte Blickachse des rechten Linsensystemkanals schneiden.

Video-Endoskope, bei denen das an einer distalen Spitze eines Endoskopschafts eintretende Licht durch ein optisches System auf einen Bildsensor oder mehrere Bildsensoren gelenkt wird, sind in verschiedenen Ausführungen bekannt. Es gibt Endoskope mit Geradeausblick, einer sogenannten 0°-Blickrichtung, Endoskope mit seitlicher Blickrichtung sowie Endoskope mit verstellbarer Blickrichtung (auch als V-DOV-Endoskope bezeichnet).

Außerdem sind Stereo-Videoendoskope bekannt, die dazu eingerichtet sind, ein stereoskopisches Bildpaar bzw. zwei stereoskopische Videokanäle aufzunehmen. Mit solchen Instrumenten ist es möglich, eine 3D-Abbildung eines distal vor dem Ende des Endoskopschafts liegenden Untersuchungs- oder Operationsraums zu erzeugen. Bei Stereo-Videoendoskopen werden die beiden optischen Kanäle aus leicht unterschiedlicher Blickrichtung aufgenommen. Der linke Kanal blickt in Richtung einer linken Blickachse, welche einen Winkel mit einer rechten Blickachse anschließt, in deren Richtung der rechte Kanal blickt. Die linke und die rechte Blickachse treffen sich in einem Achsenkreuzungspunkt im Objektraum. Dieser Punkt entspricht beim Menschen dem Kreuzungspunkt, an dem sich die Blicklinien der beiden menschlichen Augen treffen.

Die beiden Kanäle des Stereo-Videoendoskops sind durch eine Stereobasis voneinander beabstandet, gegeneinander versetzt oder verschoben. Der rechte und der linke Bildkanal werden gleichzeitig aufgenommen und über speziell geeignete Wiedergabegeräte, beispielsweise auf einem 3D-Bildschirm oder über eine 3D-Videobrille, einem Benutzer zur Verfügung gestellt. Dieser betrachtet ein 3D-Abbild der in dem Untersuchungs- oder Operationsfeld vorhandenen Objekte.

Aus DE 10 2013 215 422 A1 ist ein optisches System eines Stereo-Videoendoskops mit seitlicher Blickrichtung bekannt. Das System umfasst eine seitwärtsblickende distale optische Baugruppe. Diese ist hinter einem Eintrittsfenster angeordnet, welches den Endoskopschaft gegenüber einem Außenraum abschließt. Die distale optische Baugruppe umfasst - betrachtet in Lichteinfallsrichtung - aufeinanderfolgend eine Eintrittslinse, eine optische Ablenkeinheit und eine Austrittslinse. Auf die Austrittslinse der distalen optischen Baugruppe folgen weiter in Lichteinfallsrichtung betrachtet ein linker und ein rechter Linsensystemkanal einer proximalen optischen Baugruppe. Die beiden Linsensystemkanäle weisen jeweils eine eigene optische Achse auf und sind dazu eingerichtet, den linken und den rechten Kanal auf einen linken und einen rechten Bildsensor abzubilden. Das optische System umfasst eine einzige gemeinsame distale optische Baugruppe und zwei diskrete proximale optische Baugruppen, nämlich den linken und den rechten Linsensystemkanal.

Aus DE 10 2013 217 449 A1 ist ein weiteres optisches System eines Stereo-Videoendoskops bekannt. Dieses System erlaubt eine variable Blickrichtung. Eine Prismeneinheit dient der Veränderung der Blickrichtung, die sowohl um eine vertikale als auch um eine horizontale Achse geschwenkt werden kann. Die beiden Drehachsen stehen senkrecht auf einer Längsachse des Endoskopschafts. Die Prismeneinheit umfasst eine zentrale Umlenkeinheit und zwei Paare von Umlenkprismen, die jeweils an einander gegenüberliegenden Seiten der zentralen Umlenkeinheit angeordnet sind. Ähnlich wie bei dem aus DE 10 2013 215 422 A1 bekannten optischen System ist wiederum eine einzige und gemeinsame distale optische Baugruppe dazu eingerichtet, das aus dem Objektraum in das optische System einfallende Licht in den linken und in den rechten Linsensystemkanal einzukoppeln.

Moderne Stereo-Videoendoskope bieten hohe Bildauflösungen. Ihre Bildsensoren haben eine große Anzahl von Pixeln und entsprechend eine sehr kleine Pixelgröße. Die optischen Systeme solcher Instrumente haben Optiken (Objektive) mit großer Blendenöffnung, also einer geringen F-Zahl. Diese Optiken bieten jedoch nur eine kleine Schärfentiefe. So ist es erforderlich, die Abbildungsoptiken des linken und rechten Linsensystemkanals auf einen besten Arbeitspunkt zu fokussieren, so dass ein im Objektraum befindliches Objekt scharf auf den Bildsensoren des linken und rechten Linsensystemkanals abgebildet wird. Dieser beste Arbeitspunkt wird auch als Bestarbeitspunkt bezeichnet.

Es ist eine Aufgabe der Erfindung, ein optisches System eines Stereo-Videoendoskops, ein Stereo-Videoendoskop sowie ein Verfahren zum Betreiben eins optischen Systems eines Stereo-Videoendoskops anzugeben, wobei eine verbesserte 3D-Darstellung möglich sein soll.

Die Aufgabe wird gelöst durch ein optisches System eines Stereo-Videoendoskops, umfassend eine distale optische Baugruppe und eine proximale optische Baugruppe, wobei die proximale optische Baugruppe einen linken Linsensystemkanal mit einer linken optischen Achse und einen rechten Linsensystemkanal mit einer rechten optischen Achse umfasst, und wobei der linke und der rechte Linsensystemkanal gleichartig aufgebaut und die linke und die rechte optische Achse parallel zueinander ausgerichtet sind, und wobei die distale optische Baugruppe dazu eingerichtet ist, aus einem Objektraum einfallendes Licht in den linken Linsensystemkanal und in den rechten Linsensystemkanal der proximalen optischen Baugruppe einzukoppeln, und wobei die distale optische Baugruppe einen Achsenkreuzungspunkt im Objektraum festlegt, in dem sich eine linke Blickachse des linken Linsensystemkanals und eine rechte Blickachse des rechten Linsensystemkanals schneiden, wobei das optische System dadurch fortgebildet ist, dass die distale optische Baugruppe eine optische Baugruppe mit veränderlicher Brennweite ist, wobei eine Veränderung der Brennweite der distalen optischen Baugruppe eine Verschiebung des Achsenkreuzungspunkts im Objektraum in einer Richtung entlang einer optischen Achse der distalen optischen Baugruppe bewirkt.

Die Veränderung der Brennweite der distalen optischen Baugruppe erfolgt ähnlich oder vergleichbar dem Zoomvorgang bei einem Zoomobjektiv, wie es aus der Fotografie bekannt ist. Solche Objektive werden auch als Vario-Objektiv, Vario-System, Vario-Fokus oder Transfokar bezeichnet. Die Verschiebung des Achsenkreuzungspunkts im Objektraum erfolgt insbesondere parallel zu der optischen Achse der distalen optischen Baugruppe. Ferner ist insbesondere vorgesehen, dass die distale optische Baugruppe im Betrieb des optischen Systems aus dem Objektraum einfallendes Licht in den linken Linsensystemkanal und in den rechten Linsensystemkanal der proximalen optischen Baugruppe einkoppelt.

Das optische System des Stereo-Videoendoskops gemäß Aspekten der Erfindung beruht auf den folgenden Überlegungen.

Die Fokussierung des linken Linsensystemkanals und des rechten Linsensystemkanals erlaubt eine scharfe Abbildung von im Objektraum befindlichen Objekten. Bei diesem Vorgang bleibt jedoch die Nullebene der 3D-Abbildung unverändert - obwohl die jeweils scharf abgebildeten Objekte unterschiedlich weit von der distalen Spitze des Endoskops entfernt sind. Mit anderen Worten kann der Achsenkreuzungspunkt, an dem sich die Blickachsen des linken Linsensystemkanals und des rechten Linsensystemkanals kreuzen, sowohl vor als auch hinter derjenigen Ebene liegen, in der Objekte scharf auf den Bildsensoren der Linsensystemkanäle abgebildet werden.

In der 3D-Darstellung des von einem solchen Objekt erzeugten Bildes führt dieser Effekt dazu, dass das 3D-Bild sowohl vor als auch hinter der Oberfläche des Bildschirms zu liegen scheint. Idealerweise liegt die Nullebene der 3D-Darstellung jedoch in der Ebene der Bildschirmoberfläche. Die Verschiebung der 3D-Darstellung in Tiefenrichtung wird vom Benutzer häufig als unangenehm und anstrengend zu betrachten empfunden.

Mithilfe des optischen Systems gemäß Aspekten der Erfindung ist es durch Veränderung der Brennweite der distalen optischen Baugruppe möglich, über eine Verschiebung des Achsenkreuzungspunkts eine Verschiebung der 3D-Nullebene der 3D-Darstellung zu erreichen. Die Nullebene kann auf eine beliebige Position gelegt werden. Der Benutzer entscheidet frei und richtet sich das System so ein, dass er/sie die 3D-Darstellung als angenehm zu betrachten empfindet. Insbesondere ist es möglich, die 3D-Nullebene in eine Ebene zu legen, in der sich die Oberfläche des Monitors erstreckt.

Die mit dem optischen System gemäß Aspekten der Erfindung mögliche 3D-Darstellung ist angenehmer zu betrachten. Die Arbeit mit einem solchen System ist weniger ermüdend. Dieser Aspekt ist für ein Stereo-Videoendoskop von großer Bedeutung und hoher Relevanz. Ein Endoskop dient schließlich einem Operateur als technisches Hilfsmittel. Dieser ist bei seiner Arbeit allgemein einem hohen Stresslevel ausgesetzt. Die von einem chirurgischen Instrument bereitgestellte 3D-Darstellung sollte nicht auch noch dazu beitragen, diesen weiter zu erhöhen. Chirurgische Eingriffe erstrecken sich vielfach über längere Zeiträume. Es ist also besonders wichtig, dass es dem Operateur möglich ist, die 3D-Darstellung ermüdungsfrei zu betrachten.

Zur Abbildung von in einem Objektraum vorhandenen Objekten umfasst das optische System des Stereo-Videoendoskops beispielsweise einen linken Bildsensor und einen rechten Bildsensor. Die von dem linken Linsensystemkanal erzeugte Abbildung wird auf dem linken Bildsensor abgebildet. Der rechte Linsensystemkanal bildet auf dem rechten Bildsensor ab. Es handelt sich bei den Bildsensoren insbesondere um Sensoren mit hoher Auflösung, beispielsweise HD, 4K und folgende Technologien.

Gemäß einer vorteilhaften Ausführungsform ist vorgesehen, dass die distale optische Baugruppe zumindest ein verschiebbares optisches Element umfasst. Die Verschiebung dieses optischen Elements bewirkt eine Veränderung der Brennweite der distalen optischen Baugruppe. Es ist auch möglich, dass die distale optische Baugruppe mehr als ein verschiebbares optisches Element umfasst. Eine solche Gruppe von Elementen bewirkt dann beispielsweise die Veränderung der Brennweite. Neben der Veränderung der Brennweite kann die Verschiebung des Elements oder der optischen Elemente beispielsweise eine Verlagerung oder Verschiebung der Hauptebene der distalen optischen Baugruppe bewirken.

Gemäß einer weiteren Ausführungsform ist vorgesehen, dass die distale optische Baugruppe in einer Lichteinfallsrichtung aufeinanderfolgend eine Eintrittslinse, ein Umlenkprisma und eine Austrittslinse umfasst. Insbesondere ist die Austrittslinse das verschiebbare optische Element.

Mit anderen Worten werden zum Einstellen der 3D-Nullebene jeweils eine gemeinsame Linse, nämlich insbesondere die Austrittslinse der distalen optischen Baugruppe, sowie jeweils beispielsweise eine Linse in den einzelnen Linsensystemkanälen zum Scharfstellen der Abbildung verschoben. Dadurch wird neben der Schärfe der Abbildung auch die Position der Nullebene der 3D-Darstellung kontrolliert.

Gemäß einer weiteren Ausführungsform ist das optische System dadurch fortgebildet, dass die distale optische Baugruppe einen Zoomantrieb umfasst. Das optische System umfasst ferner insbesondere eine mit dem Zoomantrieb gekoppelte Steuereinheit, wobei die Steuereinheit dazu eingerichtet ist, den Zoomantrieb anzusteuern. Die Steuereinheit ist insbesondere derart eingerichtet, dass sie den Zoomantrieb so ansteuert, dass der Achsenkreuzungspunkt wahlweise auf einen Bestarbeitspunkt im Nahbereich oder auf einen Bestarbeitspunkt im Fernbereich eingestellt wird. Dies bedeutet, dass die Steuereinheit im Betrieb des optischen Systems den Zoomantrieb derart ansteuert, dass der Achsenkreuzungspunkt wahlweise auf den Bestarbeitspunkt im Nahbereich oder auf den Bestarbeitspunkt im Fernbereich eingestellt wird. Der Bestarbeitspunkt im Nahbereich und der Bestarbeitspunkt im Fernbereich liegen im Objektraum. Der Bestarbeitspunkt im Fernbereich ist weiter von der distalen optischen Baugruppe entfernt als der Bestarbeitspunkt im Nahbereich.

Der Zoomantrieb ist insbesondere mit dem verschiebbaren optischen Element gekoppelt. Es ist ebenso vorgesehen, dass der Zoomantrieb mit mehreren optischen Elementen gekoppelt ist. Der Antrieb ist zum Verschieben/Verlagern und zum Positionieren des verschiebbaren optischen Elements oder der verschiebbaren optischen Elemente eingerichtet. Die Verschiebung bewirkt, dass der Achsenkreuzungspunkt wahlweise auf den Bestarbeitspunkt im Nahbereich oder auf den Bestarbeitspunkt im Fernbereich eingestellt wird.

Es ist ferner insbesondere vorgesehen, dass der Bestarbeitspunkt im Nahbereich und der Bestarbeitspunkt im Fernbereich einen Mindestabstand voneinander einhalten. Dieser Mindestabstand beträgt beispielsweise wenige Millimeter oder auch einige Zentimeter. Der Bestarbeitspunkt im Nahbereich liegt ferner insbesondere in einem Nahbereich. Ebenso liegt der Bestarbeitspunkt im Fernbereich insbesondere in einem Fernbereich. Es ist beispielsweise vorgesehen, dass der Bestarbeitspunkt im Nahbereich an einer vorderen Grenze des Nahbereichs liegt. Ebenso ist beispielsweise vorgesehen, dass der Bestarbeitspunkt im Fernbereich an einer vorderen Grenze des Fernbereichs liegt. Eine vordere Grenze des Nah- oder Fernbereichs liegt am nächsten zu der distalen optischen Baugruppe. Ferner ist insbesondere vorgesehen, dass sich der Nahbereich und der Fernbereich teilweise überlappen. Sowohl der Nahbereich als auch der Fernbereich sind im Objektraum gelegen. Der Nahbereich ist dabei der Eintrittslinse der distalen Optik näher als der Fernbereich.

Es ist ebenso vorgesehen, dass mehr als zwei Punkte und auch mehr als zwei Bereiche vorgesehen sind. Dementsprechend ist die Steuereinheit beispielsweise dazu eingerichtet, den Achsenkreuzungspunkt auf mehrere Bestarbeitspunkte einzustellen. Es ist auch vorgesehen, dass die Steuereinheit dazu eingerichtet ist, den Achsenkreuzungspunkt stufenlos zu verstellen. Der Achsenkreuzungspunkt ist also variabel entlang einer Richtung verschiebbar, die zumindest näherungsweise parallel zu der optischen Achse der distalen Linsengruppe liegt.

Das optische System ist ferner insbesondere dadurch fortgebildet, dass der linke Linsensystemkanal zumindest ein verschiebbares linkes optisches Element und der rechte Linsensystemkanal zumindest ein verschiebbares rechtes optisches Element umfasst. Durch Verschieben des linken optischen Elements wird die Schärfe einer Abbildung eines in einem Objektraum befindlichen Objekts im linken Linsensystemkanal verändert. Durch Verschieben des rechten optischen Elements wird die Schärfe einer Abbildung des in dem Objektraum befindlichen Objekts im rechten Linsensystemkanal verändert. Die Steuereinheit ist dazu eingerichtet, einen linken oder einen rechten Objektabstandswert zu bestimmen, beispielsweise abzufragen oder zu messen, und den Zoomantrieb derart anzusteuern, dass der Achsenkreuzungspunkt als Funktion des linken oder rechten Objektabstandswerts verändert wird.

Der Objektabstandswert ist ein aus der Position des bewegbaren linken oder rechten optischen Elements im jeweiligen Linsensystem ableitbarer Parameter, der eine Entfernung kennzeichnet, in der sich das von dem jeweiligen Kanal scharf abgebildete Objekt befindet. Ein Objektabstandswert wäre bei einem fotografischen Objektiv beispielsweise die auf einem Schärfe-Einstellring angegebene Entfernung.

Der Zoomantrieb wird beispielsweise so angesteuert, dass er den Achsenkreuzungspunkt proportional zu einem solchen Objektabstandswert verschiebt. Beispielsweise wird der Achsenkreuzungspunkt stets in einer Ebene liegen, in der Objekte von dem linken Linsensystemkanal oder von dem rechten Linsensystemkanal scharf abgebildet werden. Hierzu ist es möglich, dass die Steuereinheit in Abhängigkeit des Objektabstandswerts des linken oder des rechten Linsensystemkanals angesteuert wird. Mit anderen Worten wird also die Lage des Achsenkreuzungspunkts der Schärfeebene eines der beiden Linsensystemkanäle nachgeführt. Es erfolgt also eine Kopplung des Zoomvorgangs der distalen optischen Baugruppe an die Fokussierung des bzw. der Linsensystemkanäle. Diese Kopplung erfolgt beispielsweise mechanisch. Es kann ein gemeinsamer Antrieb für die Fokussierung und für die Einstellung des Achsenkreuzungspunkts durch Veränderung der Brennweite der distalen optischen Baugruppe eingesetzt werden. Es ist ebenso vorgesehen, dass eine elektronische Kopplung über die Steuereinheit vorliegt.

Ferner ist insbesondere vorgesehen, dass der Achsenkreuzungspunkt intervallweise verschoben wird, wenn sich der Objektabstandswert ändert. Beispielsweise kann abgefragt werden, ob der Objektabstandswert in einem Nahbereich oder in einem Fernbereich liegt. Sofern der Objektabstandswert im Nahbereich liegt, wird beispielsweise die Steuereinheit derart angesteuert, dass der Achsenkreuzungspunkt auf den Bestarbeitspunkt im Nahbereich eingestellt wird. Wird festgestellt, dass die Ebene, in der ein Objekt scharf abgebildet wird, im Fernbereich liegt, so kann beispielsweise vorgesehen sein, dass der Achsenkreuzungspunkt durch entsprechende Ansteuerung des Zoomantriebs auf den Bestarbeitspunkt im Fernbereich eingestellt wird.

Die Aufgabe wird ferner gelöst durch ein Stereo-Videoendoskop, umfassend ein optisches System nach einem oder mehreren der zuvor genannten Ausführungsformen.

Auf das Stereo-Videoendoskop treffen gleiche oder ähnliche Vorteile zu, wie sie im Hinblick auf das optische System zuvor erwähnt wurden. Besonders vorteilhaft ist es, dass das Stereo-Videoendoskop eine 3D-Darstellung bereitstellt, die ermüdungsfrei betrachtet werden kann. Dieser Aspekt ist für ein chirurgisches Instrument, wie es das Stereo-Videoendoskop darstellt, besonders relevant. Stereo-Videoendoskope werden vielfach bei lang dauernden Operationen eingesetzt. Es ist für den Operateur sehr wichtig, dass ihm ein Arbeitswerkzeug zur Verfügung steht, welches es ihm erlaubt, auch über längere Zeit ermüdungsfrei zu arbeiten.

Die Aufgabe wird ferner gelöst durch ein Verfahren zum Betreiben eines optischen Systems eines Stereo-Videoendoskops, wobei das optische System eine distale optische Baugruppe und eine proximale optische Baugruppe umfasst, und wobei die proximale optische Baugruppe einen linken Linsensystemkanal mit einer linken optischen Achse und einen rechten Linsensystemkanal mit einer rechten optischen Achse umfasst, und wobei der linke und der rechte Linsensystemkanal gleichartig aufgebaut und die linke und die rechte optische Achse parallel zueinander ausgerichtet sind, und wobei die distale optische Baugruppe aus einem Objektraum einfallendes Licht in den linken Linsensystemkanal und in den rechten Linsensystemkanal der proximalen optischen Baugruppe einkoppelt, und wobei die distale optische Baugruppe einen Achsenkreuzungspunkt im Objektraum festlegt, in dem sich eine linke Blickachse des linken Linsensystemkanals und eine rechte Blickachse des rechten Linsensystemkanals schneiden, wobei das Verfahren dadurch fortgebildet ist, dass zum Abbilden von Objekten im Objektraum, welche unterschiedlich weit von der distalen optischen Baugruppe entfernt sind, eine Brennweite der distalen optischen Baugruppe verändert wird, wobei eine Veränderung der Brennweite der distalen optischen Baugruppe eine Verschiebung des Achsenkreuzungspunkts im Objektraum in einer Richtung entlang einer optischen Achse der distalen optischen Baugruppe bewirkt.

Auch auf das Verfahren zum Betreiben des optischen Systems treffen gleiche ohne ähnliche Vorteile zu, wie bereits im Hinblick auf das optische System zuvor erwähnt wurden.

Vorteilhaft ist das Verfahren dadurch fortgebildet, dass zum Verändern der Brennweite der distalen optischen Baugruppe zumindest ein optisches Element der distalen optischen Baugruppe verschoben wird.

Ferner ist insbesondere vorgesehen, dass die distale optische Baugruppe einen Zoomantrieb umfasst, wobei mit dem Zoomantrieb der Achsenkreuzungspunkt verschoben wird. Insbesondere ist vorgesehen, dass mit dem Zoomantrieb der Achsenkreuzungspunkt wahlweise auf einen Bestarbeitspunkt im Nahbereich oder auf einen Bestarbeitspunkt im Fernbereich eingestellt wird. Dabei liegt sowohl der Bestarbeitspunkt im Nahbereich als auch der Bestarbeitspunkt im Fernbereich im Objektraum, wobei der Bestarbeitspunkt im Fernbereich weiter von der distalen optischen Baugruppe entfernt ist als der Bestarbeitspunkt im Nahbereich.

Gemäß einer weiteren vorteilhaften Ausführungsform ist vorgesehen, dass der linke Linsensystemkanal zumindest ein verschiebbares linkes optisches Element und der rechte Linsensystemkanal zumindest ein verschiebbares rechtes optisches Element umfasst, wobei durch Verschieben des linken optischen Elements die Schärfe einer Abbildung eines in einem Objektraum befindlichen Objekts im linken Linsensystemkanal verändert wird und durch Verschieben des rechten optischen Elements die Schärfe einer Abbildung des in dem Objektraum befindlichen Objekts im rechten Linsensystemkanal verändert wird, und wobei ein linker oder ein rechter Objektabstandswert bestimmt wird und der Zoomantrieb derart angesteuert wird, dass sich der Achsenkreuzungspunkt als Funktion des linken oder rechten Objektabstandswerts ändert.

Insbesondere ist ferner vorgesehen, dass der Achsenkreuzungspunkt proportional zu dem linken oder dem rechten Objektabstandswert verändert wird. Es ist beispielsweise vorgesehen, dass der Achsenkreuzungspunkt durch entsprechende Ansteuerung des Zoomantriebs stufenweise verstellt wird. Hierzu wird beispielsweise abgefragt, ob sich der linke oder der rechte Objektabstandswert in einem bestimmten vorgegebenen Intervall befindet. Der Objektabstandswert wird anschließend beispielsweise auf die Mitte des Intervalls eingestellt. Befindet sich also beispielsweise der Objektabstandswert in einem Nahbereich, so wird der Zoomantrieb derart angesteuert, dass der Achsenkreuzungspunkt auf dem Bestarbeitspunkt im Nahbereich liegt. Gleiches gilt beispielsweise, wenn festgestellt wird, dass der Objektabstandswert in einem Fernbereich liegt. Dann wird der Achsenkreuzungspunkt auf den Bestarbeitspunkt im Fernbereich eingestellt.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematisch vereinfachte Darstellung eines Stereo-Videoendoskops umfassend ein optisches System,
- Fig. 2: eine schematisch vereinfachte Skizze, welche die stereoskopische Erfassung von Objekten mit einem linken und einem rechten Linsensystemkanal illustriert, ohne dass eine 3D-Nullebenenverschiebung vorgesehen ist,
- Fig. 3a, 3b: jeweils eine schematische und vereinfachte Darstellung eines optischen Systems eines Stereo-Videoendoskops, wobei das optische System auf einen Nahbereich (Fig. 3a) oder auf einen Fernbereich eingestellt ist (Fig. 3b) und
- Fig. 4a, 4b: weitere schematisch vereinfachte Ansichten eines optischen Systems, welches auf einen Nahbereich (Fig. 4a) oder auf einen Fernbereich (Fig. 4b) eingestellt ist.

In den Zeichnungen sind jeweils gleiche oder gleichartige Elemente und/oder Teile mit denselben Bezugsziffern versehen, so dass von einer erneuten Vorstellung jeweils abgesehen wird.

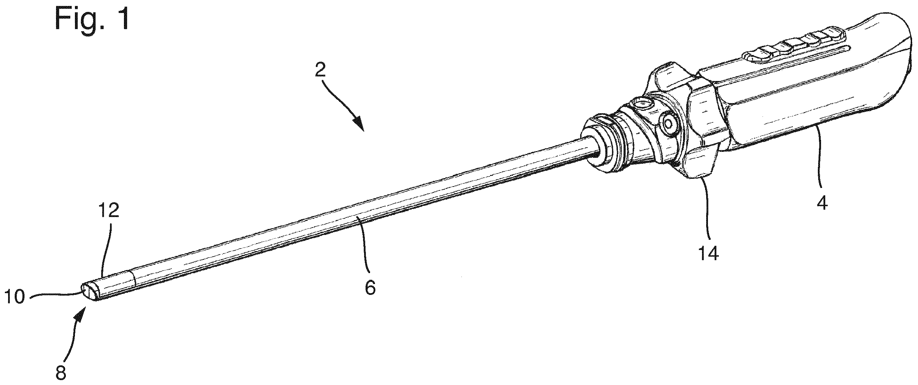

Fig. 1 zeigt in schematisch vereinfachter Darstellung ein Stereo-Videoendoskop 2 in perspektivischer Darstellung. Das Stereo-Videoendoskop 2 umfasst einen proximalen Handgriff 4, an den sich ein beispielsweise starrer Endoskopschaft 6 anschließt. An einer distalen Spitze 8 des Endoskopschafts 6 befindet sich ein Eintrittsfenster 10, durch welches Licht aus einem Operations- oder Beobachtungsfeld in ein in Fig. 1 nicht dargestelltes optisches System des Stereo-Videoendoskops 2 eintritt. Das optische System des Stereo-Videoendoskops 2 ist beispielsweise in einem distalen Abschnitt 12 des Endoskopschafts 6 angeordnet. Das optische System bildet auf diese Bildsensoren ab. Es handelt sich beispielsweise um Bildsensoren mit einer hohen Auflösung wie beispielsweise HD, 4K oder folgende Technologien.

Das gezeigte Stereo-Videoendoskop 2 kann als Instrument mit Geradeausblick (0°-Blickrichtung), mit seitlicher Blickrichtung oder mit verstellbarer Blickrichtung ausgebildet sein. Soll ein variabler oder seitlicher Blickwinkel zugelassen sein, so ist das Eintrittsfenster 10 gekrümmt und asymmetrisch ausgeführt. Eine Änderung der Blickrichtung um eine Längsachse des Endoskopschafts 6 herum wird durch eine Drehung des Handgriffs 4 um die Längsachse des Endoskopschafts 6 bewirkt. Das im distalen Abschnitt 12 vorgesehene optische System rotiert bei dieser Drehung des Handgriffs 4 mit. Zur Beibehaltung der Horizontallage des angezeigten Bildes wird bei einer Drehung des Handgriffs 4 ein Drehrad 14 festgehalten. Dies bewirkt, dass ein oder mehrere Bildsensoren im Inneren des Endoskopschafts 6 die Drehbewegung nicht mitvollziehen.

Das optische System des Stereo-Videoendoskops 2 umfasst eine distale optische Baugruppe und eine proximale optische Baugruppe. Die proximale optische Baugruppe umfasst einen linken Linsensystemkanal mit einer linken optischen Achse und einen rechten Linsensystemkanal mit einer rechten optischen Achse. Der linke und der rechte Linsensystemkanal sind gleichartig aufgebaut und die linke und die rechte optische Achse sind parallel zueinander ausgerichtet. Die distale optische Baugruppe ist dazu eingerichtet, aus dem Objektraum einfallendes Licht sowohl in den linken Linsensystemkanal als auch in den rechten Linsensystemkanal der proximalen optischen Baugruppe einzukoppeln.

Handelt es sich bei dem Stereo-Videoendoskop 2 um ein Instrument mit seitlicher Blickrichtung, so ist beispielsweise dessen optisches System, insbesondere die distale optische Baugruppe des optischen Systems, gleich oder ähnlich aufgebaut, wie es in DE 10 2013 215 422 A1 des Anmelders Olympus Winter & Ibe GmbH, Hamburg, offenbart ist. Handelt es sich bei dem Stereo-Videoendoskop 2 um ein Instrument mit variabler Blickrichtung, so ist beispielsweise dessen optisches System, insbesondere die distale optische Baugruppe, so aufgebaut, wie in DE 10 2013 217 449 A1 des Anmelders Olympus Winter & Ibe GmbH, Hamburg, offenbart. Die Offenbarung sowohl der DE 10 2013 215 422 A1 als auch der DE 10 2013 217 449 A1 sind vollumfänglich durch Bezug in die vorliegende Beschreibung aufgenommen.

Das Stereo-Videoendoskop 2 ist dazu eingerichtet, ein stereoskopisches Bildpaar bzw. zwei stereoskopische Videokanäle aufzunehmen. So ist es möglich, eine 3D-Abbildung eines in einem Untersuchungs- oder Operationsraum liegenden Objekts, welches sich distal vor der distalen Spitze 8 befindet, zu erzeugen. Mit einem geeigneten und nicht dargestellten Wiedergabegerät, beispielsweise einem 3D-Monitor oder einer 3D-Videobrille, ist es einem Benutzer des Stereo-Videoendoskops 2 möglich, ein 3D-Abbild dieses Objektes zu betrachten.

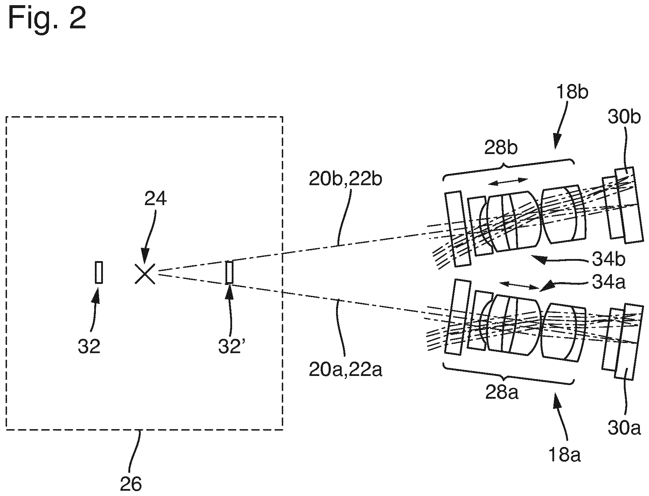

Fig. 2 zeigt eine schematisch vereinfachte Skizze, welche eine stereoskopische Erfassung von Objekten mithilfe eines linken und eines rechten Linsensystemkanals eines optischen Systems illustriert. Die Skizze in Fig. 2 stellt eine Anordnung des gezoomten rechten und linken Linsensystemkanals im optischen System des Stereo-Videoendoskops 2 dar. Die Nullebene des optischen Systems ist nicht einstellbar. Der linke und der rechte Linsensystemkanal sind in einem Winkel zueinander angeordnet. Bei dem optischen System des Stereo-Videoendoskops 2 ist dies nicht der Fall, die beiden Linsensystemkanäle sind parallel (in Bezug auf ihre optischen Achsen) zueinander angeordnet.

In der in Fig. 2 dargestellten Skizze blickt der linke Linsensystemkanal 18a in Richtung einer linken Blickachse 20a, welche mit der linken optischen Achse 22a des linken Linsensystemkanals 18a zusammenfällt. Der rechte Linsensystemkanal 18b blickt in Richtung einer rechten Blickachse 20b, welche mit der rechten optischen Achse 22b zusammenfällt. Die linke Blickachse 20a und die rechte Blickachse 20b schneiden sich in einem Achsenkreuzungspunkt 24 in einem Objektraum 26.

Bei dem optischen System des Stereo-Videoendoskops 2 sind die optischen Achsen 22a, 22b der Linsensystemkanäle 18a, 18b parallel zueinander ausgerichtet. Die mechanische Struktur sorgt dafür, dass sich die linke Blickachse 20a des linken Linsensystemkanals 18a und die rechte Blickachse 20b des rechten Linsensystemkanals 18b im Achsenkreuzungspunkt 24 im Objektraum 26 schneiden. Bei dem optischen System des Stereo-Videoendoskops 2 fallen also anders als in Fig. 2 die optischen Achsen 22a, 22b und die Blickachsen 20a, 20b nicht zusammen.

Es handelt sich bei einer linken Optik 28a und einer rechten Optik 28b, welche auf einen linken Bildsensor 30a oder auf einen rechten Bildsensor 30b abbilden und jeweils gemeinsam mit diesem den linken oder rechten Linsensystemkanal 18a, 18b bilden, um lichtstarke Optiken mit großer Blendöffnung bzw. niedriger F-Zahl. Solche Optiken 28a, 28b weisen eine geringe Tiefenschärfe auf, so dass es erforderlich ist, diese zu fokussieren, um ein unterschiedlich weit entferntes Objekt 32, 32', welches sich im Objektraum 26 befindet, jeweils nach dem Bedarf scharf auf die Bildsensoren 30a und 30b abzubilden. Hierzu umfasst die linke Optik 28a eine linke verschiebbare Linsengruppe 34a, die rechte Optik 28b umfasst entsprechend eine identische rechte verschiebbare Linsengruppe 34b.

Durch Verschieben der verschiebbaren Linsengruppe 34a und 34b ist es möglich, die Ebene, in welcher Objekte scharf auf den Sensor 30a, 30b abgebildet werden, entlang der optischen Achse 22a, 22b zu verschieben. Es ist also durch Fokussierung der linken bzw. rechten Optik 28a, 28b sowohl möglich, das Objekt 32 als auch das Objekt 32' jeweils scharf auf den Bildsensor 30a und 30b abzubilden. Beim Fokussieren bleibt der Achsenkreuzungspunkt 24, in welchem sich die linke und rechte Blickachse 20a, 20b schneiden, unverändert. Mit anderen Worten wird also ein Objekt 32 scharf abgebildet, welches hinter dem Achsenkreuzungspunkt 24 liegt oder es wird ein Objekt 32' scharf abgebildet, welches vor dem Achsenkreuzungspunkt 24 liegt. Die Begriffe "dahinter" und "davor" beziehen sich auf die Entfernung der Objekte 32, 32' betrachtet, ausgehend vom linken und vom rechten Linsensystemkanal 18a, 18b, in Bezug auf die Entfernung des Achsenkreuzungspunkts 24.

Die Abbildung von Objekten, welche vor oder hinter dem Achsenkreuzungspunkt 24 liegen, führt zu einer Verschiebung der 3D-Nullebene in der 3D-Darstellung. Das jeweils abgebildete Objekt 32, 32' scheint vor oder hinter einer Oberfläche eines Bildschirms zu liegen, welcher die 3D-Ansicht des Objekts 32, 32' bereitstellt. Eine solche 3D-Darstellung wird vielfach als unangenehm und ermüdend zu betrachten empfunden.

Um diesem Effekt entgegenzuwirken, umfasst das optische System des Stereo-Videoendoskops 2 gemäß Aspekten der Erfindung eine distale optische Baugruppe mit veränderlicher Brennweite. Durch die Veränderung der Brennweite ist es möglich, den Achsenkreuzungspunkt 24 im Objektraum 26 in Richtung einer optischen Achse der distalen optischen Baugruppe zu verschieben.

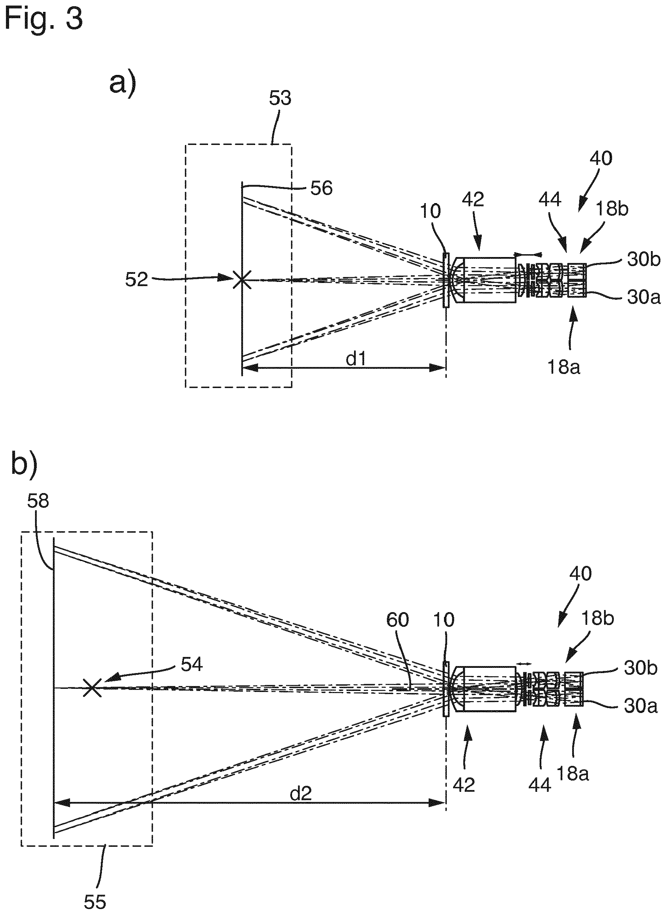

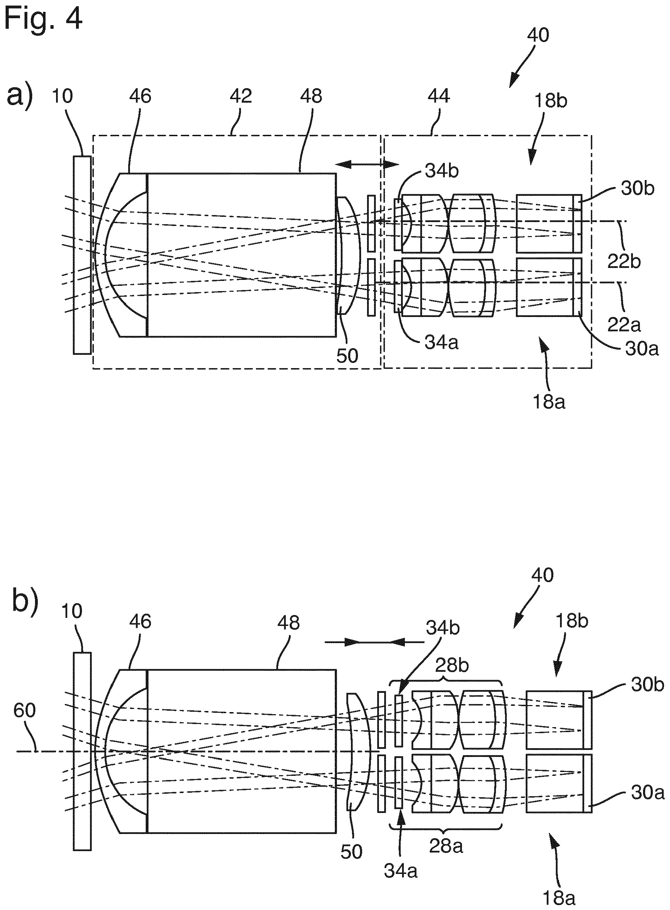

Fig. 3a und 3b zeigen jeweils eine schematische und vereinfachte Darstellung des optischen Systems 40, wobei das optische System 40 in der Darstellung von Fig. 3a auf einen Nahbereich und in der Darstellung von Fig. 3b auf einen Fernbereich eingestellt ist. Fig. 4a und 4b zeigen weitere schematisch vereinfachte Ansichten des optischen Systems 40, welches auf den Nahbereich (Fig. 4a) oder auf den Fernbereich (Fig. 4b) eingestellt ist. Das optische System 40 wird unter Bezugnahme auf die Fig. 3a, 3b, 4a und 4b beschrieben.

Das optische System 40 umfasst eine distale optische Baugruppe 42 (in Fig. 4a mit gestrichelter Linie umrandet) sowie eine proximale optische Baugruppe (in Fig. 4a mit gepunkteter Linie umrandet). Wie bereits erwähnt, umfasst die proximale optische Baugruppe 44 den linken Linsensystemkanal 18a und den rechten Linsensystemkanal 18b. Die beiden Linsensystemkanäle 18a, 18b sind so angeordnet, dass die linke optische Achse 22a des linken Linsensystemkanals 18a und die rechte optische Achse 22b des rechten Linsensystemkanals 18b parallel zueinander ausgerichtet sind (vgl. Fig. 4a). Der linke und der rechte Linsensystemkanal 18a, 18b sind außerdem gleich oder gleichartig aufgebaut. Sie umfassen jeweils eine Optik 28a, 28b (vgl. Fig. 4b), mit welcher Licht auf einen linken oder einen rechten Bildsensor 30a, 30b abgebildet wird. Zum Fokussieren umfassen die linke und die rechte Optik 28a, 28b jeweils eine verschiebbare Linsengruppe 34a, 34b bzw. eine verschiebbare Linse. Der Fokussiervorgang wird beim Vergleich der Abbildungen in Fig. 4a und 4b deutlich. Die Linsengruppe 34a, 34b befindet sich jeweils an unterschiedlicher Position.

Die distale optische Baugruppe 42 ist dazu eingerichtet, aus dem Objektraum 26 einfallendes Licht sowohl in den linken als auch in den rechten Linsensystemkanal 18a, 18b einzukoppeln. Die distale optische Baugruppe 42 ist eine optische Baugruppe mit veränderlicher Brennweite. Dies wird bewirkt durch Verschiebung eines verschiebbaren optischen Elements, welches von der distalen optischen Baugruppe 42 umfasst ist. In den dargestellten Ausführungsbeispielen umfasst die distale optische Baugruppe 42 in Lichteinfallsrichtung aufeinanderfolgend eine Eintrittslinse 46, ein Umlenkprisma 48 sowie eine Austrittslinse 50. Ferner ist in Fig. 4a und 4b das Eintrittsfenster 10 dargestellt, welches den Innenraum des Endoskopschafts 6 gegenüber einem Außenraum abschließt (Fig. 1). Die Veränderung der Brennweite der distalen optischen Baugruppe 42 erfolgt durch Verschiebung der Austrittslinse 50. Dies geht aus einem Vergleich der Fig. 4a und 4b beispielhaft hervor. Es handelt sich also bei der Austrittslinse 50 beispielhaft um das verschiebbare optische Element. Eine Veränderung der Brennweite der distalen optischen Baugruppe 42 bewirkt eine Verschiebung des Achsenkreuzungspunkts 24 im Objektraum 26. Diese Verschiebung erfolgt in einer Richtung entlang einer optischen Achse 60 der distalen optischen Baugruppe 42 (vgl. Fig. 3b und 4b).

Die distale optische Baugruppe 42 ist beispielsweise mit einem nicht dargestellten Zoomantrieb versehen oder umfasst diesen. Ferner ist in dem optischen System 40 eine mit diesem Zoomantrieb gekoppelte ebenfalls nicht dargestellte Steuereinheit vorgesehen. Die Steuereinheit ist dazu eingerichtet, den Zoomantrieb stufenlos anzutreiben oder aber bestimmte Positionen oder Einstellungen einzustellen. Insbesondere ist vorgesehen, dass die Steuereinheit den Zoomantrieb derart ansteuert, dass der Achsenkreuzungspunkt 24 auf einem Bestarbeitspunkt im Nahbereich 52 (vgl. Fig. 3a) oder auf einem Bestarbeitspunkt im Fernbereich 54 (vgl. Fig. 3b) liegt.

Der Bestarbeitspunkt im Nahbereich 52 und der Bestarbeitspunkt im Fernbereich 54 liegen im Objektraum 26. Wie ein Vergleich der Fig. 3a und 3b ergibt, liegt der Bestarbeitspunkt im Fernbereich 54 weiter von der distalen optischen Baugruppe 42 entfernt als der Bestarbeitspunkt im Nahbereich 52. Beispielsweise wird in diesem Zusammenhang Bezug auf eine Entfernung zwischen dem Eintrittsfenster 10 und der jeweiligen Lage des Nah- bzw. Bestarbeitspunkt im Fernbereichs 52, 54 genommen. Der Bestarbeitspunkt im Nahbereich 52 liegt in einem Nahbereich 53. Der Bestarbeitspunkt im Fernbereich 54 liegt in einem Fernbereich 55. Die distale optische Baugruppe 42 ist beispielsweise so eingerichtet, dass ihr verschiebbares optisches Element, also beispielsweise die Austrittslinse 50, in zwei verschiedenen Positionen positionierbar ist, so dass der Achsenkreuzungspunkt 24 wahlweise auf dem Bestarbeitspunkt im Nahbereich 52 oder auf dem Bestarbeitspunkt im Fernbereich 54 liegt.

Zur Abbildung von Objekten, welche in dem Nahbereich 53 liegen, liegen die Objektebene, in der Objekte scharf abgebildet werden, und der Achsenkreuzungspunkt, welcher auf dem Bestarbeitspunkt im Nahbereich 52 liegt, nah genug beieinander, so dass keine nennenswerten Abweichungen der 3D-Nullebene für den Benutzer wahrnehmbar ist. Werden Objekte abgebildet, die im Fernbereich 55 liegen, so wird der Achsenkreuzungspunkt 24 auf den Bestarbeitspunkt im Fernbereich 54 eingestellt. Erneut können Objekte in dem Fernbereich 55 scharf abgebildet werden, ohne dass eine nennenswerte Abweichung der 3D-Nullebene beispielsweise von einer Oberfläche des Bildschirms für den Benutzer wahrnehmbar ist. Beispielsweise liegt der Bestarbeitspunkt im Nahbereich 52 in der Mitte des Nahbereiches und der Bestarbeitspunkt im Fernbereich 54 liegt in der Mitte des Fernbereichs 55.

Abweichend von der Darstellung in Fig. 3 können der Nahbereich 53 und der Fernbereich 55 so eingerichtet sein, dass diese unmittelbar aneinander anschließen. Dann wäre kein Abstand zwischen dem Nahbereich 53 und dem Fernbereich 55 vorgesehen.

Die Einstellung der distalen optischen Baugruppe 42 auf den Nahbereich 53 oder den Fernbereich 55 erfolgt beispielsweise, indem ein Objektabstandswert von dem rechten oder dem linken Linsensystemkanal 18a, 18b abgefragt wird/werden. Der Objektabstandswert ist diejenige Entfernung, in der sich Objekte befinden, die scharf auf den jeweiligen Sensor 30a, 30b abgebildet werden. In der Darstellungen von Fig. 3a sind dies Objekte in der nahen Objektebene 56, also mit Objektabstandswert d1. In Fig. 3b sind dies Objekte, die in der fernen Objektebene 58 liegen. Diese haben den Objektabstandswert d2. Der Objektabstandswert d1, d2 wird beispielsweise zwischen der Ebene 56, 58 und dem Eintrittsfenster 10 gemessen.

Wird beispielsweise von der Steuereinheit des optischen Systems 40 festgestellt, dass der Objektabstandswert d1, d2 in dem Nahbereich 53 liegt, so wird veranlasst, dass die distale optische Baugruppe 42 den Achsenkreuzungspunkt 24 auf den Bestarbeitspunkt im Nahbereich 52 einstellt. In Fig. 3b ist die ferne Objektebene 58 um den zweiten Objektabstandswert d2 von dem Eintrittsfenster 10 beabstandet. Stellt die Steuereinheit des optischen Systems 40 fest, dass der zweite Objektabstandswert d2 innerhalb des Fernbereichs 55 liegt, so wird die distale optische Baugruppe 42 so eingestellt, dass der Achsenkreuzungspunkt 24 auf dem Bestarbeitspunkt im Fernbereich 54 liegt.

Mit anderen Worten ist also der Zoomantrieb der distalen optischen Baugruppe 42 dazu eingerichtet, den Achsenkreuzungspunkt 24 als Funktion des Objektabstandswertes d1, d2 zu verändern.

Es ist neben dieser stufenweisen Veränderung auch beispielsweise vorgesehen, dass der Achsenkreuzungspunkt 24 kontinuierlich der Lage der scharf abgebildeten Objektebene 56, 58 nachgeführt wird. In diesem Fall würde also die Brennweite der distalen optischen Baugruppe 42 beispielsweise proportional zum Wert des Objektabstandswerts verändert. Der Objektabstandswert d1, d2, auf dessen Grundlage der Achsenkreuzungspunkt 24 nachgeführt wird, wird beispielsweise anhand eines von dem linken oder von dem rechten Linsensystemkanal 18a, 18b gelieferten Objektabstandswerts bestimmt.

Bei einem Verfahren zum Betreiben des optischen Systems 40 eines Stereo-Videoendoskops 2 wird zum Abbilden von Objekten 32, 32' im Objektraum 26, welche unterschiedlich weit von der distalen optischen Baugruppe 42 entfernt sind, eine Brennweite der distalen optischen Baugruppe 42 verändert. Die Veränderung der Brennweite bewirkt eine Verschiebung des Achsenkreuzungspunkts 24 im Objektraum 26 in einer Richtung entlang einer optischen Achse 60 der distalen optischen Baugruppe 42 (vgl. Fig. 4b). Durch die Verschiebung bzw. Nachführung des Achsenkreuzungspunkts 24 in Bezug auf eine Objektebene 56, 58, in der Objekte scharf abgebildet werden, wird eine 3D-Darstellung bereitgestellt, deren Nullebene stets nah an einer Bildschirmoberfläche bzw. einer Nullebene des Wiedergabegeräts liegt.

Abweichend von dieser Nachführung ist es auch möglich, den Achsenkreuzungspunkt 26 zur gezielten Verschiebung der 3D-Nullebene frei einzustellen. Der Benutzer wird in die Lage versetzt, die 3D-Darstellung so einzustellen, dass ihm ein möglichst angenehmes und ermüdungsfreies Arbeiten möglich ist.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: Stereo-Videoendoskop
- 4: Handgriff
- 6: Endoskopschaft
- 8: distale Spitze
- 10: Eintrittsfenster
- 12: distaler Abschnitt
- 14: Drehrad
- 18a: linker Linsensystemkanal
- 18b: rechter Linsensystemkanal
- 20a: linke Blickachse
- 20b: rechte Blickachse
- 22a: linke optische Achse
- 22b: rechte optische Achse
- 24: Achsenkreuzungspunkt
- 26: Objektraum
- 28a: linke Optik
- 28b: rechte Optik
- 30a: linker Bildsensor
- 30b: rechter Bildsensor
- 32, 32': Objekt
- 34a: linke verschiebbare Linsengruppe
- 34b: rechte verschiebbare Linsengruppe
- 40: optisches System
- 42: distale optische Baugruppe
- 44: proximale optische Baugruppe
- 46: Eintrittslinse
- 48: Umlenkprisma
- 50: Austrittslinse
- 52: Bestarbeitspunkt im Nahbereich
- 53: Nahbereich
- 54: Bestarbeitspunkt im Fernbereich
- 55: Fernbereich
- 56: nahe Objektebene
- 58: ferne Objektebene
- 60: optische Achse der distalen optischen Baugruppe

- d1,d2: Objektabstandswert

## Patentansprüche

1. Optisches System (40) eines Stereo-Videoendoskops (2), umfassend eine distale optische Baugruppe (42) und eine proximale optische Baugruppe (44), wobei die proximale optische Baugruppe (44) einen linken Linsensystemkanal (18a) mit einer linken optischen Achse (22a) und einen rechten Linsensystemkanal (18b) mit einer rechten optischen Achse (22b) umfasst, und wobei der linke und der rechte Linsensystemkanal (18a, 18b) gleichartig aufgebaut und die linke und die rechte optische Achse (22a, 22b) parallel zueinander ausgerichtet sind, und wobei die distale optische Baugruppe (42) dazu eingerichtet ist, aus einem Objektraum (26) einfallendes Licht in den linken Linsensystemkanal (18a) und in den rechten Linsensystemkanal (18b) der proximalen optischen Baugruppe (44) einzukoppeln, und wobei die distale optische Baugruppe (42) einen Achsenkreuzungspunkt (24) im Objektraum (26) festlegt, in dem sich eine linke Blickachse (22a) des linken Linsensystemkanals (18a) und eine rechte Blickachse (22b) des rechten Linsensystemkanals (18b) schneiden, **dadurch gekennzeichnet, dass** die distale optische Baugruppe (42) eine optische Baugruppe mit veränderlicher Brennweite ist, wobei eine Veränderung der Brennweite der distalen optischen Baugruppe (42) eine Verschiebung des Achsenkreuzungspunkts (24) im Objektraum (26) in einer Richtung entlang einer optischen Achse (60) der distalen optischen Baugruppe (42) bewirkt.

2. Optisches System (40) nach Anspruch 1, **dadurch gekennzeichnet, dass** die distale optische Baugruppe (42) zumindest ein verschiebbares optisches Element umfasst, wobei die Verschiebung des verschiebbaren optischen Elements die Veränderung der Brennweite der distalen optischen Baugruppe (42) bewirkt.

3. Optisches System (40) nach Anspruch 2, **dadurch gekennzeichnet, dass** die distale optische Baugruppe (42) in einer Lichteinfallsrichtung aufeinanderfolgend eine Eintrittslinse (46), ein Umlenkprisma (48) und eine Austrittslinse (50) umfasst, wobei die Austrittslinse (50) das verschiebbare optische Element ist.

4. Optisches System (40) nach einem der Ansprüche 1 bis 3, **dadurch gekennzeichnet, dass** die distale optische Baugruppe (42) einen Zoomantrieb umfasst und das optische System (40) eine mit dem Zoomantrieb gekoppelte Steuereinheit umfasst, wobei die Steuereinheit dazu eingerichtet ist, den Zoomantrieb derart anzusteuern, dass der Achsenkreuzungspunkt (24) wahlweise auf einen Bestarbeitspunkt im Nahbereich (52) oder auf einen Bestarbeitspunkt im Fernbereich (54) eingestellt wird, wobei der Bestarbeitspunkt im Nahbereich (52) und der Bestarbeitspunkt im Fernbereich (54) im Objektraum (26) liegen und der Bestarbeitspunkt im Fernbereich (54) weiter von der distalen optischen Baugruppe (42) entfernt ist als der Bestarbeitspunkt im Nahbereich (52).

5. Optisches System (40) nach Anspruch 4, **dadurch gekennzeichnet, dass** der linke Linsensystemkanal (18a) zumindest ein verschiebbares linkes optisches Element und der rechte Linsensystemkanal (18b) zumindest ein verschiebbares rechtes optisches Element umfasst, wobei durch Verschieben des linken optischen Elements die Schärfe einer Abbildung eines in einem Objektraum (26) befindlichen Objekts (32, 32') im linken Linsensystemkanal (18a) verändert werden kann und durch Verschieben des rechten optischen Elements die Schärfe einer Abbildung des in dem Objektraum (26) befindlichen Objekts (32, 32') im rechten Linsensystemkanal (18b) verändert werden kann, und wobei die Steuereinheit dazu eingerichtet ist, einen linken oder einen rechten Objektabstandswert (d1, d2) zu bestimmen und den Zoomantrieb derart anzusteuern, dass der Achsenkreuzungspunkt (24) als Funktion des linken oder rechten Objektabstandswerts (d1, d2) verändert wird.

6. Stereo-Videoendoskop (2), **gekennzeichnet durch** ein optisches System (40) nach einem der Ansprüche 1 bis 5.

7. Verfahren zum Betreiben eines optischen Systems (40) eines Stereo-Videoendoskops (2), wobei das optische System (40) eine distale optische Baugruppe (42) und eine proximale optische Baugruppe (44) umfasst, und wobei die proximale optische Baugruppe (44) einen linken Linsensystemkanal (18a) mit einer linken optischen Achse (22a) und einen rechten Linsensystemkanal (18b) mit einer rechten optischen Achse (22b) umfasst, und wobei der linke und der rechte Linsensystemkanal (18a, 18b) gleichartig aufgebaut und die linke und die rechte optische Achse (22a, 22b) parallel zueinander ausgerichtet sind, und wobei die distale optische Baugruppe (42) aus einem Objektraum (26) einfallendes Licht in den linken Linsensystemkanal (18a) und in den rechten Linsensystemkanal (18b) der proximalen optischen Baugruppe (44) einkoppelt, und wobei die distale optische Baugruppe (42) einen Achsenkreuzungspunkt (24) im Objektraum (26) festlegt, in dem sich eine linke Blickachse (20a) des linken Linsensystemkanals (18a) und eine rechte Blickachse (20b) des rechten Linsensystemkanals (18b) schneiden, **dadurch gekennzeichnet, dass** zum Abbilden von Objekten (32, 32') im Objektraum (26), welche unterschiedlich weit von der distalen optischen Baugruppe (42) entfernt sind, eine Brennweite der distalen optischen Baugruppe (42) verändert wird, wobei eine Veränderung der Brennweite der distalen optischen Baugruppe (42) eine Verschiebung des Achsenkreuzungspunkts (24) im Objektraum (26) in einer Richtung entlang einer optischen Achse (60) der distalen optischen Baugruppe (42) bewirkt.

8. Verfahren nach Anspruch 7, **dadurch gekennzeichnet, dass** zum Verändern der Brennweite der distalen optischen Baugruppe (42) zumindest ein optisches Element der distalen optischen Baugruppe (42) verschoben wird.

9. Verfahren nach Anspruch 7 oder 8, **dadurch gekennzeichnet, dass** die distale optische Baugruppe (42) einen Zoomantrieb umfasst, wobei mit dem Zoomantrieb der Achsenkreuzungspunkt (26) wahlweise auf einen Bestarbeitspunkt im Nahbereich (52) oder auf einen Bestarbeitspunkt im Fernbereich (54) eingestellt wird, und wobei der Bestarbeitspunkt im Nahbereich (52) und der Bestarbeitspunkt im Fernbereich (54) im Objektraum (26) liegen und der Bestarbeitspunkt im Fernbereich (54) weiter von der distalen optischen Baugruppe (42) entfernt ist als der Bestarbeitspunkt im Nahbereich (52).

10. Verfahren nach Anspruch 9, **dadurch gekennzeichnet, dass** der linke Linsensystemkanal (18a) zumindest ein verschiebbares linkes optisches Element und der rechte Linsensystemkanal (18b) zumindest ein verschiebbares rechtes optisches Element umfasst, wobei durch Verschieben des linken optischen Elements die Schärfe einer Abbildung eines in einem Objektraum (26) befindlichen Objekts (32, 32') im linken Linsensystemkanal (18a) verändert wird und durch Verschieben des rechten optischen Elements die Schärfe einer Abbildung des in dem Objektraum (26) befindlichen Objekts (32, 32') im rechten Linsensystemkanal (18b) verändert wird, und wobei ein linker oder ein rechter Objektabstandswert (d1, d2) bestimmt wird und der Zoomantrieb derart angesteuert wird, dass sich der Achsenkreuzungspunkt (24) als Funktion des linken oder rechten Objektabstandswerts (d1, d2) ändert.
